# EUROPEAN PATENT APPLICATION

(11) **EP 3 722 263 A1**
(43) Date of publication of application: **14.10.2020**
(21) Application number: 19168501.5
(22) Date of filing: 10.04.2019
(51) Int. Cl.: C02F 3/34, C12N 9/02

(54) **WASTEWATER TREATMENT USING BACILLUS SUBTILUS**

(71) Applicant: Amapex Environment SL, 08029 Barcelona (ES)
(72) Inventor: Canadell Casanova, Joaquim, 08029 Barcelona (ES); Canyes Sala, Antoni, 08029 Barcelona (ES); Cañas Sala, Jordi, 08029 Barcelona (ES)
(74) Representative: Brantsandpatents bvba

(57) **Abstract**

In a first aspect, the current invention concerns a biological wastewater treatment method for organochlorine (OC) polluted wastewaters, the method comprising the degradation of OC compounds using a Bacillus subtilis strain, whereby said *B. subtilis* strain is able to produce a laccase with a sufficient enzymatic activity against said OC compounds. In a further aspect, the invention concerns the use of the *B. subtilis* IAB/BS03 strain deposited at the DSMZ German Type Culture Collection with accession number DSM 24682 or a mutant thereof, for a treatment of wastewaters, preferably OC polluted wastewaters.

## Description

### Technical field

The invention pertains to the technical field of biological wastewater treatments. More in particular the invention pertains to a wastewater treatment for the degradation of organochlorine (OC) compounds.

### Background

Bioremediation is any process that uses microorganisms, fungi, green plants or their enzymes for treating a contamination of the natural environment, mostly caused by wastewater. More particularly, bioremediation uses living systems especially microorganisms to catalyze the degradation of recalcitrant chemicals without disrupting the environment. The main chemical constituents in wastewater, released from industrial sectors such as the chemical industry and agriculture, include but are not limited to organochlorine (OC) compounds. In particular OC pesticides are released.

OC pesticides are synthetic pesticides widely used all over the world. They belong to the group of chlorinated hydrocarbon derivatives. These compounds are known for their high toxicity, slow degradation and bioaccumulation. Even though many of said compounds were banned in developed countries, the use of these agents has been rising. This concerns particularly abuse of these chemicals which is in practice across the continents. About 40% of all pesticides used belong to the OC class of chemicals (Gupta, 2004; FAO, 2005). Due to their low cost and their need against various pests, OC insecticides such as DDT, hexachlorocyclohexane (HCH), aldrin and dieldrin are among the most used pesticides around the world (FAO, 2005; Gupta, 2004; Lallas, 2001).

The use of laccase to degrade OC compounds has been proposed. Laccase is a copper containing polyphenol oxidase that catalyzes the redox reaction of phenolic compounds and plays an important role in the biodegradation of lignin and its precursor analogs. Laccase has a wide range of oxidizing substrates, including phenols and their derivatives, aromatic amines and their derivatives and aromatic carboxylic acids and their derivatives. However, laccase can also oxidize chlorophenolic compounds and its derivatives, reduce its toxicity and reduce the environmental pollution caused by the production of dyes, preservatives, herbicides, pesticides and other chemical products using chlorophenols as industrial raw material.

Heterologous expression of laccase has often been used as a strategy to get around the problem of obtaining laccases that are not easily producible in natural hosts. However, very often the production yield is low, due to a strong tendency of this enzyme to form aggregates which renders the protein irreversibly inactive. This tendency has been attributed to the fact that in nature laccase is integrated in a spore coat structure via interaction with other protein components, and it is likely that correct laccase folding is enhanced by interaction with other proteins. When this laccase is recombinantly expressed as an individual polypeptide, those supporting interactions are missing and many miss-folded proteins form aggregates in bacterial cells. When expressed in higher microorganisms such as yeast, misfolded laccase molecules are degraded for a large part.

CN 103 320 374 pertains to a genetic engineered bacterium for the high-efficiency expression of a laccase enzyme native to *B*. *subtilis.* The invention pertains to a recombinant microorganism (MO). CN '374 discloses using the genetic engineered bacterium in a wastewater treatment. In particular in a wastewater treatment for printing and dyeing waste streams. The invention does not pertain to the degradation of OC compounds.

CN 106 045 004 pertains to a process for the catalytic degradation of a chlorine bleaching effluent using a laccase enzyme and a metal salen complex. CN '004 discloses using a laccase enzyme in wastewater treatments. The degradation of pesticides and/or insecticides is not disclosed. The invention has the disadvantage that laccase has to be produced separately.

CN 102 115 722 pertains to a preparation method of a laccase enzyme using a *B*. *subtilis* strain. CN '722 discloses using the laccase enzyme in wastewater treatments. In particular wastewater treatments for industrial dye waste streams and industrial pulp and paper waste streams. The bacteria are not directly added to the wastewater treatment. The laccase enzyme is separately produced using a fermentation.

There is a need in the art for natural hosts that easily produce laccases. There is also a need in the art for means and methods for improving the yield of laccases. This is particularly true for bacterial laccases. Moreover, there is a need in the art for wastewater treatment means and methods that are able to efficiently degrade OC compounds, in particular OC pesticides.

The present invention aims to resolve at least some of the technical problems mentioned above.

### Summary of the invention

In a first aspect, the invention relates to a biological wastewater treatment method for organochlorine (OC) polluted wastewaters according to claim 1. The method comprises the degradation of OC compounds using a Bacillus subtilis strain, whereby said *B*. *subtilis* strain is able to produce a laccase with a sufficient enzymatic activity against said OC compounds.

In a second aspect, the invention relates to a doser unit comprising an incubator according to claim 16. The incubator, comprised by the doser unit, is suited for preparing an activated mixture according to the current disclosure.

In a third aspect, the invention relates to the use of the *B*. *subtilis* IAB/BS03 strain or a mutant thereof for treatment of wastewaters according to claim 18. The *B*. *subtilis* IAB/BS03 strain is deposited at the DSMZ German Type Culture Collection with accession number DSM 24682.

The invention has great commercial applications in textile plants, pulp and paper manufacturing units, oil refineries, petrochemical plants, fermentation plants, pharmaceuticals, tanneries, chemical manufacturing, food processing plants and any other unit discharging recalcitrant OC compounds in wastewater. In particular the invention has a great commercial applicability for the degradation of OC polluted wastewaters.

### Detailed description of the invention

The present invention concerns a biological wastewater treatment method for organochlorine (OC) polluted wastewaters. The present invention also concerns a doser unit comprising an incubator, wherein said incubator is suited for preparing an activated mixture. The present invention furthermore concerns the use of the *B*. *subtilis* IAB/BS03 strain for treatment of wastewaters. In what follows, the invention is described in detail, preferred embodiments are discussed and the invention is illustrated via examples.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

In a first aspect, the invention is related to a biological wastewater treatment method for organochlorine (OC) polluted wastewaters. The method comprises the degradation of OC compounds using a *Bacillus subtilis* strain, whereby said *B. subtilis* strain is able to produce a laccase with a sufficient enzymatic activity against said OC compounds.

"Enzymatic activity" within the confines of this text refers to the ability of an enzyme to act as a catalyst in a process, such as the conversion of one compound to another compound.

"Sufficient enzymatic activity" within the confines of this text should be understood as an enzyme that has a substantially detectable enzymatic activity against a specific substrate, which can be measured by detecting a product or by-product of the enzymatic reaction. The detectable activity may be the binding of the active molecule to the substrate, which can be measured by detecting the bound complex or the amount of free active molecule or substrate. It is particularly preferred, enzymes having a "sufficient enzymatic activity" for a particular function are those that are able to degrade at least 50 % of the total amount of substrate, preferably at least about 60 %, preferably at least about 70 %, more preferably at least about 80 %> and most preferably at least about 85 %, as measured using an art-recognized assay for the particular function of interest.

In a preferred embodiment of the invention said enzymatic activity comprises a dehalogenation of the OC compounds. Preferably, wherein at least about 50 % of the total amount of OC compounds is dehalogenated, more preferably at least about 60 %, more preferably at least about 70 %, more preferably at least about 80 %> and most preferably at least about 85 %.

Preferably said laccase has at least 0.5 units of dehalogenase activity, more preferably said laccase has at least 1 unit of dehalogenase activity and most preferably said laccase has at least 1.5 units of dehalogenase activity.

"Units" should be understood as the ability to convert a specific content of a compound to a different compound under a defined set of conditions as a function of time. Preferably, 1 "unit" of dehalogenase activity relates to the ability to convert 1 pmol of tetrachloroethylene to its corresponding alkene or alkane per minute, per milligram of cells (dry weight) at a of pH 7.0 and a temperature of 30° C.

The invention has great commercial applications in textile plants, pulp and paper manufacturing units, oil refineries, petrochemical plants, fermentation plants, pharmaceuticals, tanneries, chemical manufacturing, food processing plants and any other unit discharging recalcitrant OC compounds in wastewater.

In an embodiment of the invention said *B. subtilis* strain is able to produce other enzymes with a sufficient enzymatic activity against said OC compounds. Preferably said *B. subtilis* strain is able to produce proteases and/or lipases with a sufficient enzymatic activity against said OC compounds.

Preferably the OC compounds are chlorinated pesticides or insecticides. Said compounds are known for their high toxicity, slow degradation and bioaccumulation. Moreover, said compounds result in a particular recalcitrant COD. Degrading such compounds has great commercial applications.

Preferably the OC compounds comprise one or more compounds selected from the group: chlorophenols, chlorinated paraffins, hexachlorocyclohexanes, tetrachloroethylene, or derivatives thereof. Such compounds result in a particular recalcitrant COD. Degrading such compounds has great commercial applications.

In a preferred embodiment of the invention, the OC polluted wastewater further comprises a hydrocarbon (HC) pollution. Preferably, at least 50 % of said HC pollution originates from industrial lubricants and/or refrigerants, more preferably at least 60 % and most preferably at least 70%. The biological wastewater treatment method comprises at least a 75% efficient phase-separation of the HC pollution and an aqueous phase, preferably at least an 85% efficient phase-separation and most preferably at least a 95% efficient phase-separation

Emulsions comprising a HC pollution are very stable and thus difficult to separate from an aqueous phase. Industrial lubricants and/or refrigerants are in particular difficult to separate. In general, an additional treatment plant is needed for said separation. As the current invention allows for an at least 75% efficient phase-separation of the HC pollution and an aqueous phase, a simple gravity filtration system suffices.

In a preferred embodiment of the invention, the method comprises a step of preparing an activated mixture by incubating the *B. subtilis* strain in a substrate comprising sodium molybdate (Na₂MoO₄), Copper(II) Copper Sulphate (CuSO4) and Magnesium (II) Manganesium Sulphate (MnSO4).

"Activated mixture" within the confines of this text should be understood as a mixture comprising a substantial volume percentage of the laccase, a substantial volume percentage of the *B. subtilis* strain and a substantial volume percentage of the substrate. Preferably the activated mixture comprises about 3%-15% v/v of laccase, more preferably of about 5%-12% v/v, most preferably 7%-10% v/v. Preparing an activated mixture has the advantage that the *B. subtilis* strain is incubated in an optimal growth and/or production environment.

Preferably the laccase is extracellularly excreted.

"Extracellularly excreted" within the confines of this text should be understood as the excretion of a protein that is expressed in a bacterium to the periplasm or extracellular growth medium.

There are several potential advantages to having a laccase secreted into the periplasmic space/extracellular medium, including the avoidance of cytoplasmic proteases, the prevention of any toxic effect on the host, an easier purification of the laccase and the ability to prepare an activated mixture according to the current invention.

In an embodiment of the invention, the *B. subtilis* strain comprises an expression vector comprising secretory signal sequence and an inducible promoter suited for extracellularly excreting said laccase.

Prior to incubation, the *B. subtilis* strain is cultivated in pure culture till the logarithmic phase of growth. About 5 to 15% (v/v) of the pure culture is then taken for preparation of the inoculants. Preferably the inoculants is further incubated for a minimum time period of at least 24 hours. Said minimum time period is required to achieve maximum growth of the *B. subtilis* strain for preparation of the activated mixture. By separately incubating the *B. subtilis* strain the inoculants concentration, the incubation time and the incubation temperature can be adjusted in order to achieve the desired ratio of volume percentage of laccase, *B. subtilis* strain and substrate.

In an embodiment of the invention, the *B. subtilis* strain is incubated at a temperature of about 37 to 45 °C. Preferably the *B. subtilis* strain is incubated at a temperature of about 43 °C. Traditionally, reproduction is inhibited and growth is erratic for *B. subtilis* strains cultivated at such temperatures. The inventors unexpectedly observed an exponential growth and laccase production at such temperatures.

In a further embodiment of the invention, the *B. subtilis* strain is incubated at a Dissolved Oxygen (DO) concentration at least 2 mg/L. Preferably the *B. subtilis* strain is incubated at a DO concentration of at least 4 mg/L. The inventors observed that such DO concentrations are required in order to maintain the exponential growth and laccase production at the elevated temperatures.

In an embodiment of the invention, the substrate further comprises one or more nutrients selected from the group: yeast extract, peptic digest of animal tissue, di-ammonium phosphate and sodium chloride, tryptone, soya extract, enzymatic digest of gelatin, magnesium sulphate, calcium chloride, magnesium chloride and potassium sulphate. Preferably the substrate comprises one or more nutrients selected from the group: 0.2%-0.5% of yeast extract, 0.3-0.6% peptic digest of animal tissue, 0.05%-0.2% of di-ammonium phosphate and 0.3%-0.8% of sodium chloride, 0.3%-0.6% of tryptone, 0.2%-0.5% of soya extract, 0.2%- 0.5% of enzymatic digest of gelatin , 0.3%-0.8% of magnesium sulphate, 0.3%-0.8% of calcium chloride, 0.3%-0.8% of magnesium chloride and 0.3%-0.8% of potassium sulphate.

In an embodiment of the invention, the method comprises providing a sensor configured to measure the potential of hydrogen (pH), the conductivity, and the temperature of the activated mixture.

In a preferred embodiment of the invention, the method further comprises a step of adding the activated mixture to OC polluted wastewaters. The combination is allowed to react with the wastewater. The activated mixture for the removal of OC pollutants is a synergistic composition due to which the treatment of wastewater for removal of OC pollutants is very effective and reduces the total time that is required for such processes.

In an embodiment of the invention, the method comprises a step of storing the activated mixture in a storage tank prior to adding the mixture to OC polluted wastewaters. In a further embodiment of the invention, the method further comprises providing a sensor in the storage tank, wherein said sensor is configured to measure the pH, the conductivity, and the temperature of the activated mixture in the storage tank. Such methodology has the advantage that the mixture is stabilized.

Preferably the activated mixture is stored at a temperature of about 20 to 30 °C. Storing the activated mixture at such temperature ensures a short re-activation time of the *B. subtilis* strain upon addition to the OC polluted wastewaters. Moreover, such temperature stabilizes the enzymatic activity of the laccase.

For example, in an embodiment of the invention, the desired activity of the enzyme, or the microorganism capable of producing the enzyme, is stabilized such that the desired activity after a time of at least 30 days at a temperature of about 25° C is maintained at a level of at least about 50% of the initial activity exhibited by the enzyme or the microorganism capable of producing the enzyme.

In a preferred embodiment of the invention, the activated mixture is added to OC polluted wastewaters by dosing the activated mixture based on a OC content of said wastewaters. Such methodology allows for a proper dosing and incubation period of the activated mixture.

In an embodiment of the invention, the method further comprises providing a sensor configured to measure the pH, the conductivity, and the temperature of the OC polluted wastewater.

In another embodiment of the invention, the chemical oxygen demand (COD) of the OC polluted wastewaters is determined prior to the treatment by open reflux method using potassium dichromate. In this method, a fixed volume with known concentration of potassium dichromate is added to suitably diluted wastewater sample. After a refluxing digestion step with concentrated sulphuric acid the initial concentration of organic substances in the sample is calculated from a titrimetric or spectrophotometric determination of the oxidant still remaining in the sample. A blank sample is created by adding all reagents (e.g. acid and oxidizing agent) to a volume of distilled water. COD is measured for both the water and blank samples, and the two are compared. The oxygen demand in the blank sample is subtracted from the COD for the original sample to ensure a true measurement of organic matter.

In a further embodiment of the invention, the method further comprises a step of adding enzymes to the OC polluted wastewaters. Preferably the enzymes are selected from peroxidases (manganese dependent and manganese independent), lignin peroxidase, laccase, catalase, cytochrome c oxidase, glucose oxidase, phenol oxidase, n- and o-demethylase, protease, lipase, alpha-amylase and bacteriocin alone or in combination. More preferably the enzymes are selected from 10%-20% v/v of peroxidases (manganese dependent and manganese independent), 7%-10% v/v of lignin peroxidase, 7%- 10% v/v of laccase, 1%-5% v/v of catalase, 0.5%-3% v/v of cytochrome c oxidase, 5%-10% v/v of glucose oxidase, 3%-5%v/v of phenol oxidase, 1%-2% v/v of n-and o-demethylase, 5%-7% v/v of protease, 5%-7% v/v of lipase, 5% v/v of alpha-amylase and 1% v/v of bacteriocin.

By adding enzymes in combination with the activated mixture to the OC polluted wastewaters, the composition exhibits synergy and more effectively removes the pollutants from the wastewater. Enzymes dissociate the pollutant molecules to simpler form and microbes utilize these simple intermediates in their metabolic activities thereby completely degrading the pollutants in the wastewater. Microbes grow faster due to the increasing availability of simple intermediates and produce more enzymes which can further degrade the pollutants.

In a second aspect, the invention is related to a doser unit comprising an incubator, wherein said incubator is suited for preparing an activated mixture according to the current disclosure.

In a preferred embodiment of the invention, the doser unit further comprises a storage tank configured with a dosing means suited for adding the activated mixture to OC polluted wastewaters according to the current disclosure.

All the above-mentioned advantages and effects related to incubating and storing a *B. subtilis* strain can be repeated with respect to the doser unit comprising an incubator.

In another aspect, the present invention is related to the use of the *B. subtilis* IAB/BS03 strain deposited at the DSMZ German Type Culture Collection with accession number DSM 24682 or a mutant thereof, for a treatment of OC polluted wastewaters.

In a preferred embodiment of the method, the invention relates to a wastewater treatment method, wherein the *B. subtilis* strain is the *B. subtilis* IAB/BS03 strain deposited at the DSMZ German Type Culture Collection with accession number DSM 24682, or a mutant thereof.

All the above-mentioned advantages and effects related to a *B. subtilis* strain can be repeated with respect to the *B. subtilis* IAB/BS03 strain.

This written description uses examples to disclose embodiments, including the preferred mode, and also to enable any person skilled in the art to make and use such embodiments. While various specific embodiments have been described, those skilled in the art will recognize other embodiments can be practiced with modification within the scope of the claims. Especially, mutually non-exclusive features of the embodiments described above may be combined with each other. The patentable scope is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

The invention is further described by the following non-limiting examples which further illustrate the invention, and are not intended to, nor should they be interpreted to, limit the scope of the invention.

### Exam pies

### Example 1: Feasibility study

In general, industries that have a wastewater treatment plant usually combine two or more technologies. The combination of the most appropriate technologies is selected depending on the type of contaminants, their load, previous use of the wastewater and economic affordability. Physical, chemical and biological technologies are combined in different stages of treatment: preliminary, primary, secondary and tertiary. The most commonly used technologies for the treatment of wastewaters were analyzed during the feasibility study.

An example of a pre-treatment technology is decantation. Decantation removes large constituents from wastewater, such as rags, sticks, floatables or gritt. Preventing maintenance and operational problems during further steps of the wastewater treatment. The primary treatment technologies comprise: coagulation-flocculation, Fat separation and Sand filtration. Said technologies remove important residues which require an additional treatment to be removed. The secondary treatment technologies comprise biological treatments. This is a classic secondary treatment which uses a wide variety of MOs, primarily bacteria. These MOs convert biodegradable organic matter into simple substances and additional biomass, but they usually reduce only 40% of fat and organic matter, which does not allow for water recycling. Tertiary treatment technologies comprise activated carbon for absorption, Reverse osmosis membranes and ozonization. Said technologies are only used for treatment of small volumes of effluents due to the high cost.

The current wastewater treatment systems of different sectors were analyzed and compared to a wastewater treatment system using the *B. subtilis* IAB/BS03 strain according to the current invention. An overview of the currently used technologies by the different sectors is shown in the table below. An X indicates that the technology is used in the sector.

| | Textile | Tanning | Carwash stations | Metal mechanic | OC Compounds |
|---|---|---|---|---|---|
| Decantation | X | x | X | X | X |
| Coagulation/flocculation | X | X | | | X |
| Fat separation | | X | X | X | |
| Anaerobic oxidation | X | X | | X | X |
| Aerobic hydrolysis | | | | | X |
| Biological membrane | | | X | X | |
| Sand filtration | X | X | | X | |
| Active carbon | X | X | | X | X |
| Ozone | X | | | X | X |
| Membranes | | X | | X | X |
| Inverted osmosis | | X | | X | X |

The ability to reduce the COD of the currently used technologies was compared to a wastewater treatment system using the *B. subtilis* IAB/BS03 strain according to the current invention. More in particular a wastewater treatment system wherein an activated mixture was prepared according to the conditions as described by the current invention. An overview of the effectiveness of the current invention compared to the different sectors is shown in the table below. The effectiveness is express in percentage of COD removed compared to the influent of the wastewater treatment plant.

| | Textile | Tanning | Carwash stations | Metal mechanic | Chemical |
|---|---|---|---|---|---|
| Current wastewater treatment systems | | | | | |
| Internal treatment cost (€/m³) | 8.0 | 55.0 | 20.0 | 69.0 | 110.0 |
| Effectiveness (%) | 20 | 90 | 20 | 70 | 100 |
| Internal weighted cost (€/m³) | 40.0 | 61.1 | 100.0 | 98.6 | 110.0 |

| Wastewater treatment system using the *B. subtilis* IAB/BS03 strain | | | | | |
|---|---|---|---|---|---|
| Internal treatment cost (€/m³) | 15.0 | 40.0 | 25.0 | 45.0 | 45.0 |
| Effectiveness (%) | 83 | 92 | 95 | 95 | 100 |
| Internal weighted cost (€/m³) | 18.1 | 43.5 | 26.3 | 47.4 | 45.0 |
| Cost reduction (%) | 10 | 29 | 42 | 52 | 59 |

As illustrated by the table above, the current concept results in a cost reduction of about 10 to 59% depending on the sector. The end quality of the effluent is also higher compared to the current wastewater treatment systems. This higher quality allows for the reuse of the effluent. This reuse is not possible using the current wastewater treatment systems and up to 60% of the water costs can be saved depending on the sector. It is important to note that a wastewater treatment according to the current concept is also able to replace the entire set of secondary and tertiary processes of the current wastewater treatment systems.

### Example 2: Insecticide degradation

Hexachlorocyclohexane (HCH) rich effluent from an insecticide producing plant was used for the study. The pH of the effluent was adjusted to 7 with HCI. For test sample, the effluent was pre-treated using a decantation and underwent a coagulation-flocculation step. Subsequently hydrogen peroxide and ferrous sulphate were added to 500 ml of waste water sample which was kept on magnetic stirrer for 30 minutes to facilitate proper mixing and diffusion.

An activated mixture was prepared by inoculating a *B. subtilis* strain in a substrate comprising CuSO₄, MnSO₄ and Na₂MoO₄. The strain was incubated for 27 hours at a temperature of about 43 °C and a DO concentration of about 4 mg/L. An activated mixture with a 12% v/v laccase concentration was obtained. 1 ml of this combination was added to the waste water sample and kept on a magnetic stirrer for 30 minutes at 30°C and 50 rpm. The control sample consisted of a waste water sample without the added activated mixture. The control sample was also heated to 30 °C and stirred at 50 rpm.

5 ml samples were collected from both tests as well as from the control sets at 0 h, 6h, 12h and 24h from the start of the experiment. The samples were centrifuged at 5000 rpm for 5 minutes. The HCH content of the supernatant was estimated by RP-HPLC on a column with methanol:water 60:40 as mobile and detected by a UV detector at 254 nm. The results are shown in the table below.

| Time (hours) | Estimated HCH content | | | | | |
|---|---|---|---|---|---|---|
| | Set 1 (Control) | | | Set 2 (Test) | | |
| | Peak area (mAU) | HCH content (ppm) | % Reduction | Peak area (mAU) | HCH content (ppm) | % Reduction |
| 0 | 153 | 12 | 0 | 153.2 | 12 | 0 |
| 6 | 152.1 | 11.9 | 0.01 | 102 | 8 | 33.3 |
| 12 | 151 | 11.8 | 0.18 | 85 | 6.6 | 45 |
| 24 | 152 | 11.9 | 0.01 | 20 | 1.6 | 87 |

There was a significant reduction in HCH content in the test sample, whereas no significant reduction was observed in the control sample. Indicating the specificity of the treatment method towards OC compounds. In particular towards HCH. Similar results were found for chlorophenols, chlorinated paraffins and tetrachloroethylene.

### Example 3: Trace element study

An activated mixture was prepared by inoculating a *B. subtilis* strain in a substrate. The strain was subsequently incubated for 27 hours at a temperature of about 43 °C and a DO concentration of 4 mg/L. Three test sample according to the procedure above were prepared. The differentiating factor between the three samples is the substrate that was used.

Test sample 1 was prepared using a substrate comprising CuSO₄, MnSO₄ and Na₂MoO₄. The substrate of test sample 2 was depleted of its CoCl₂ content. The substrate of test sample 3 was depleted of its Na₂MoO₄ content. The substrate of test sample 4 was depleted of its Na₂MoO₄ content. Test sample 5 was depleted of its CuSO₄, MnSO₄ and Na₂MoO₄ content. The growth of the *B. subtilis* strain and the laccase production were analyzed. 3 ml samples were collected from all test samples at 0 h, 6h, 12h and 24h from the start of the experiment. The results are shown in the table below.

| Time (hour s) | Productivity | | | | | | | | | |
|---|---|---|---|---|---|---|---|---|---|---|
| | Test sample 1 | | Test sample 2 | | Test sample 3 | | Test sample 4 | | Test sample 5 | |
| | %v/v laccase concentration | %v/v dry weight cells | %v/v laccase concentration | %v/v dry weight cells | %v/v laccase concentration | %v/v dry weight cells | %v/v laccase concentration | %v/v dry weight cells | %v/v laccase concentration | %v/v dry weight cells |
| 0 | 0 | 5 | 0 | 5 | 0 | 5 | 0 | 5 | 0 | 5 |
| 6 | 7 | 21 | 0 | 15 | 0 | 14 | 0 | 11 | 0 | 9 |
| 12 | 9 | 43 | 0 | 39 | 1 | 38 | 0 | 35 | 0 | 25 |
| 24 | 14 | 67 | 1 | 62 | 1 | 61 | 0 | 59 | 0 | 45 |

There was a significant increase in laccase content in test sample 1 compared to the other Test samples. No laccase production was observed for test samples 4 and 5. Similarly, a slight increase in growth was observed for test sample 1 compared to the other test samples. Which indicates a synergistic effect of CuSO₄, MnSO₄ and Na₂MoO₄ on both the growth and laccase production.

It is supposed that the present invention is not restricted to any form of realization described previously and that some modifications can be added to the presented example of fabrication without reappraisal of the appended claims.

## Claims

1. A biological wastewater treatment method for organochlorine (OC) polluted wastewaters, the method comprising the degradation of OC compounds using a *Bacillus subtilis* strain, whereby said *B. subtilis* strain is able to produce a laccase with a sufficient enzymatic activity against said OC compounds.

2. The method according to claim 1, wherein said enzymatic activity comprises a dehalogenation of the OC compounds.

3. The method according to claim 2, wherein said laccase has at least 0.5 units of dehalogenase activity.

4. The method according to one of the preceding claims 1 to 3, wherein the OC polluted wastewaters further comprise a hydrocarbon (HC) pollution and wherein the method further comprises an at least 75% efficient phase-separation of the HC pollution and an aqueous phase.

5. The method according to claim 4, wherein the HC pollution comprises industrial lubricants and/or refrigerants.

6. The method according to any of the preceding claims 1 to 5, wherein the method comprises a step of preparing an activated mixture by incubating said *B. subtilis* strain in a substrate comprising sodium molybdate (Na₂MoO₄), Copper (II) sulphate (CuSO₄) and magnesium (II) sulphate (MnSO₄).

7. The method according to claim 6, wherein the method further comprises a step of adding the activated mixture to OC polluted wastewaters.

8. The method according to any of the preceding claims 6 or 7, wherein the *B. subtilis* strain is incubated at a temperature of about 43 °C.

9. The method according to any of the preceding claims 6 to 8, wherein the *B. subtilis* strain is incubated at a Dissolved Oxygen (DO) concentration of at least 2 mg/L.

10. The method according to any of the preceding claims 7 to 9, wherein the activated mixture is added to OC polluted wastewaters by dosing the activated mixture based on an OC content of said wastewaters.

11. The method according to any of the preceding claims 1 to 10, wherein the laccase is extracellularly excreted.

12. The method according to any of the preceding claims 1 to 11, wherein the *B. subtilis* strain is the *B. subtilis* IAB/BS03 strain deposited at the DSMZ German Type Culture Collection with accession number DSM 24682, or a mutant thereof.

13. A doser unit comprising an incubator, wherein said incubator is suited for preparing an activated mixture according to any of the preceding claims 6 to 12.

14. The doser unit according to claim 13, wherein the doser unit further comprises a storage tank configured with a dosing means suited for adding the activated mixture to OC polluted wastewaters according to any of the preceding claims 7 to 13.

15. Use of the *B. subtilis* IAB/BS03 strain deposited at the DSMZ German Type Culture Collection with accession number DSM 24682 or a mutant thereof, for treatment of wastewaters, preferably OC polluted wastewaters.
